# EUROPEAN PATENT SPECIFICATION

(11) **EP 1 779 877 B1**
(45) Date of publication and mention of the grant of the patent: **15.12.2010**
(21) Application number: 06255075.1
(22) Date of filing: 29.09.2006
(51) Int. Cl.: A61L 27/16, A61L 27/50, A61L 27/54

(54) **Bearing materials for medical implants**
Gelenkeoberflächematerialen zur Herstellung von medizinische Implantate
Matériaux pour paliers de prothèses implantables

(30) Priority: 30.09.2005 US 241136
(43) Date of publication of application: 02.05.2007
(73) Proprietor: DePuy Products, Inc., Warsaw, IN 46581 (US)
(72) Inventor: King, Richard S., Warsaw, IN 46582 (US); Hanes, Mark D., Winona Lake, IN 46590 (US)
(74) Representative: Belcher, Simon James

(56) References cited:
- EP-A- 1 493 775
- US-A1- 5 827 904

## Description

This invention relates to a bearing material for a medical implant or implant part, and to a method of making a bearing material.

US-5827904 relates to a polymer composition for use in a medical implant. The composition is doped with a carotenoid in order to increase oxidation resistance. The doping is carried out by immersing the polymeric material in a solution of the carotenoid in isopropyl alcohol. EP-A-1493775 discloses a polymeric implant in which ultrahigh molecular weight polyethylene (UHMWPE) is immersed in a hydrophobic liquid so that the liquid is absorbed into the UHMWPE.

Implants have been used to replace parts of the human body, e.g., the hip, the knee, and the extremity joints. Post-surgery pain and infection are some of the medical concerns in orthopaedic joint replacements. Accordingly, attempts have been made to deliver a beneficial agent such as a drug by the use of coated implants wherein the coating includes a drug. For example, implants coated with a polyurethane coating or polymethyl methacrylate bone cement coating have been used in orthopaedic applications to deliver drugs to the site of pain or infection. These implants, however, have one or more drawbacks. There is a desire to improve upon one or more properties of such implants, particularly the beneficial agent release profile. The present invention provides such an implant.

In an implant, the bearing material having a bearing surface is paired with an opposing metal or ceramic component. The bearing surface is also called the articulating surface. The invention provides a bearing material for a medical implant or medical implant part, the bearing material comprising: (a) a matrix of crosslinked polyethylene, (b) a biocompatible hydrophobic carrier, and (c) a beneficial agent soluble in the biocompatible hydrophobic carrier. An advantage of the bearing material of the invention is that the beneficial agent release profile is at least partially load activated. The beneficial agent release mechanism, unlike the prior art devices, is not purely passive. The beneficial agent release from the bearing material of the invention can be based on a combination of load-activated and passive mechanisms. Cyclic loading of the bearing material during use pumps out the hydrophobic carrier and beneficial agent and provides a sustained release of the agent without significantly compromising its mechanical properties, for example, wear resistance.

The invention also provides methods for preparing a bearing material in accordance with the invention. The methods rely on soaking polyethylene in a solution of the beneficial agent in a biocompatible hydrophobic carrier. The biocompatible hydrophobic carrier and the beneficial agent are diffused into the polyethylene. The methods of the invention have one or more of the following advantages. Unlike methods involving melt processing which could lead to degradation, especially when processing high melting and/or thermally sensitive beneficial agents or carriers, it is possible by the methods of the present invention to process with significantly reduced degradation of the beneficial agent or the biocompatible carrier. The methods of the invention also provide more homogeneous or uniform distribution, e.g., improved uniformity of distribution of the carrier and beneficial agent, compared to methods which mould a blend of powdered polyethylene, carrier, and beneficial agent. The methods of the invention do not require the use of solvents which are not biocompatible, e.g., solvents such as isopropyl alcohol, cyclohexane, n-hexane, and benzene.

Accordingly, the present invention provides a bearing material as defined in claim 1 and a method for producing said bearing material as defined in claim 6.

In accordance with the present invention, any suitable polyethylene can be used. UHMWPE has excellent wear resistance for various modes of loading. The normal shear and compressive loading on UHMWPE articulating surface provides a mechanism for delivery of a beneficial agent to the patient. The UHMWPE can be non-crosslinked, or preferably crosslinked.

The term "ultrahigh molecular weight polyethylene" refers to a polyethylene polymer having a weight average molecular weight of about 0.4 × 10⁶ amu or more. Preferably, the UHMWPE has a weight average molecular weight of at least about 10⁶ (e.g., at least about 2 × 10⁶ or at least about 3 × 10⁶) amu. Typically, the weight average molecular weight of the UHMWPE is not more than about 10⁷ amu, more preferably not more than about 6 × 10⁶ amu. UHMWPE materials which are suitable for use in the invention include commercially available UHMWPEs such as GUR 1050 and GUR 1020 powdered UHMWPE (weight average molecular weight of about 2 × 10⁶ to about 6 × 10⁶ amu) from Ticona (Summit, New Jersey).

In accordance with the invention, any suitable biocompatible hydrophobic carrier can be used, for example, the biocompatible hydrophobic carrier is selected from the group consisting of squalane, squalene, benzyl benzoate, fatty acids, glycerides, polyisoprenoids, cholesterol, cholesterol esters, and any combination thereof, particularly squalane, squalene, and benzyl benzoate. Preferably, the biocompatible hydrophobic carrier is a lipid, e.g., those selected from the group consisting of squalane, squalene, fatty acids, glycerides, polyisoprenoids, cholesterol, cholesterol esters, and any combination thereof, particularly squalane and squalene. In accordance with the invention, the bearing material includes the biocompatible hydrophobic carrier in an amount from 0.1 wt.% to 10 wt.%, and more preferably from about 1 wt.% to about 5 wt.% of the bearing material.

A drug is any chemical compound that affects the processes of the human mind or body, for example, a compound used on or administered to humans or animals as an aid in the diagnosis, treatment, or prevention of disease or other abnormal condition, for the relief of pain or suffering, or to control or improve any physiologic or pathologic condition. Any suitable beneficial agent can be employed, e.g., those selected from the group consisting of antibiotics, analgesics, anti-bone resorption drugs, bone growth factors, anti-cancer drugs, antioxidants, and any combination thereof. Examples of chemicals include antioxidants. Antioxidants could reduce or prevent the oxidation and degradation of the articulating surface of the bearing material. Examples of biologicals include a substance made from a living organism or its products. Biologicals may be used to prevent, diagnose, treat or relieve of symptoms of a disease. Examples of biologicals include antibodies, interleukins, and immunomodulators.

The drug has a solubility of at least about 1 % by weight in the hydrophobic carrier, and generally of from about 1% to about 100% or more, and preferably from about 10% to about 100% by weight of the hydrophobic carrier. The beneficial agent can be included in the bearing material in any suitable amount, for example, in an amount of from about 0.001 wt.% to about 20 wt.%, preferably from about 0.01 wt.% to about 10 wt.%, and more preferably from about 0.1 wt.% to about 5 wt.% of the bearing material.

The orthopaedic bearing material of the invention can be prepared by any suitable method, e.g., by diffusing a solution of the beneficial agent in a biocompatible hydrophobic carrier into a polyethylene. For example, an irradiated polyethylene can be soaked in a solution of the beneficial agent. In accordance with an embodiment, the invention provides a method as defined in claim 6.

The raw material in consolidated form is a precursor to the bearing material, which can be of any consolidated shape, e.g., a rod, sheet, preform, or a finished part. The raw material in consolidated form can be prepared by any suitable method, for example, by moulding, extrusion, or solvent casting. Alternatively, the raw material in consolidated form can be machined or moulded from a block or sheet of a polymer, e.g., of a crosslinkable polymer such as UHMWPE.

Irradiation can be carried out by using any suitable radiation, e.g., ionizing radiation. Ionizing radiation is a radiation, in which an individual particle, e.g., electron, positron, alpha particle, or neutron, carries high enough energy, or an electromagnetic radiation having a high enough energy, to ionize an atom or molecule in the irradiated substrate. Examples of electromagnetic radiation include gamma radiation, X-ray, and ultraviolet light, preferably gamma radiation.

The raw material in consolidated form can be exposed to any suitable amount or dose of radiation, such as from about 1 to about 100 Mrad or more, preferably from about 5 to about 25 Mrad, and more preferably from about 5 to about 10 Mrad. The energy of the radiation is selected so that it is effective to crosslink at least a portion of the exposed surface of the raw material in consolidated form of the bearing material.

Optionally, the raw material in consolidated form after irradiation (or matrix), can be treated suitably so that any free radicals generated during irradiation are reduced or eliminated. An example of such a treatment is heat treatment, e.g., remelting or annealing. Remelting involves heating the irradiated polyethylene above its melting temperature, e.g., from about 1°C to about 160°C above the melting temperature of the irradiated polyethylene, preferably from about 40°C to about 80°C above the melting temperature. Thus, for example, the remelting temperature for irradiated UHMWPE can be from about 136°C to about 300°C, preferably from about 136°C to about 250°C, and more preferably from about 13b°C to about 200°C.

Generally, the remelting temperature is inversely proportional to the remelting period. Polyethylene is preferably remelted over a period from about 1 hour to about 2 days, more preferably from about 1 hour to about 1 day, and most preferably from about 2 hours to about 12 hours. Since, depending on the time and temperature applied, annealing can produce less of an effect than remelting on physical properties such as crystallinity, yield strength and ultimate strength, annealing may be used in place of remelting as a means for reducing or eliminating the free radicals remaining in the polymer after irradiation crosslinking, in order to maintain the physical properties within limits required by the user. Thermal treatment, such as remelting or annealing, removes free radicals and thereby improves long term wear resistance of the polymer.

Annealing involves heating the crosslinked polyethylene below its melting temperature. The annealing temperature can be from about room temperature to below the melting temperature of the irradiated polyethylene; preferably from about 90 ° C to about 1°C below the melting temperature of the irradiated polymer; and more preferably from about 60°C to about 1°C below the melting temperature of the irradiated polyethylene. For example, irradiated UHMWPE may be annealed at a temperature from about 25°C to 135°C, preferably from about 50°C to about 135°C, and more preferably from about 80°C to about 135°C. The annealing period can be from about 2 hours to about 7 days, preferably from about 7 hours to about 5 days, and more preferably from about 10 hours to about 2 days.

The solution comprising the drug and hydrophobic carrier can optionally include other suitable additives, for example, surfactants, solubilising agents such as co-solvents or complexing agents, viscosity modifiers, swelling agents, and stabilizing agents.

The irradiated matrix, or portion thereof, for example at least about 10%, at least about 20%, at least about 30%, at least about 40%, at least about 50%, at least about 60%, at least about 70%, at least about 80%, at least about 90%, or about 100 percent of the matrix, can be contacted with the solution in any suitable method, for example, the matrix is soaked or immersed in the solution. The solution can be maintained at any suitable temperature, for example, at a temperature of at least about 30°C, preferably at least about 50°C, more preferably at least about 60°C. The temperature will frequently be not more than about 150°C, preferably not more than about 120°C, and more preferably not more than about 110°C.

The irradiated matrix can be contacted with the solution for any suitable period of time, for example, for a period of from about 0.1 hour or more, such as for about 2 hours or more, preferably from about 8 to about 24 hours. The contact time can vary with the temperature, for example, inversely with temperature. The amount of solution absorbed will depend on the temperature, contact time, sample size, and surface area.

The matrix produced in the method can be dried to remove excess biocompatible hydrophobic carrier or therapeutic agent and yield a bearing material having a desired final concentration of biocompatible hydrophobic carrier and beneficial agent.

Optionally, the raw material in consolidated form, e.g., rod, sheet, or preform, can be shaped by suitable method, e.g., machining, a shape and size desired for the bearing material. This can be performed before or after irradiation. The orthopaedic bearing material can be sterilized by a suitable method, e.g., by a non-irradiative method such as the use of ethylene oxide gas.

In another embodiment, irradiation can be carried out after diffusing the beneficial agent and carrier into the raw material in consolidated form.

The raw material in consolidated form, the solution comprising a biocompatible hydrophobic carrier and a beneficial agent, contacting conditions, irradiating conditions, and other treatments are as described above, except that the raw material in consolidated form is contacted with the solution comprising the beneficial agent and the biocompatible hydrophobic carrier first followed by irradiation of the thus contacted raw material in consolidated form.

The raw material in consolidated form can be dried, e.g., under vacuum, to remove excess biocompatible hydrophobic carrier or beneficial agent and yield a raw material in consolidated form having a desired concentration of biocompatible hydrophobic carrier and the agent.

It is contemplated that the bearing material of the invention can have a number of uses. For example, the bearing material can be a prosthetic acetabular cup, an insert or liner of the acetabular cup, a trunnion bearing or a component thereof, a prosthetic tibial plateau, a patellar button, a prosthetic talar surface, a prosthetic radio-humeral joint, an ulno-humeral joint, a glenoro-humeral articulation, an intervertebral disk replacement, a facet joint replacement, a temporo-mandibular joint, or a finger joint. The bearing material may find use in the hip, knee, and extremity joints. The bearing material can be a liner for the acetabular component of a hip arthroplasty or the tibial bearing for a knee arthroplasty.

The orthopaedic bearing material or implant of the invention, can find use as a prosthesis for any suitable part of the body, e.g., such as a component of a joint in the body. For example, in a hip joint, the orthopaedic bearing material or implant can be a prosthetic acetabular cup, or the insert or liner of the cup, or a component of a trunnion bearing (e.g., between the modular head and the stem). In a knee joint, the orthopaedic bearing material or implant can be a prosthetic tibial plateau (femoro-tibial articulation), a patellar button (patello-femoral articulation), a trunnion or other bearing component, depending on the design of the artificial knee joint. For example, in a knee joint of the meniscal bearing type, both the upper and lower surfaces of the orthopaedic bearing material or implant, i.e., those surfaces that articulate against metallic or ceramic surfaces, may be surface-crosslinked. In an ankle joint, the orthopaedic bearing material or implant can be the prosthetic talar surface (tibio-talar articulation) or other bearing component. In an elbow joint, the orthopaedic bearing material or implant can be the prosthetic radio-humeral joint, the ulno-humeral joint, or other bearing component. In a shoulder joint, the orthopaedic bearing material or implant can be used in the glenoro-humeral articulation. In the spine, the orthopaedic bearing material or implant can be used in intervertebral disk replacement or facet joint replacement. The orthopaedic bearing material or implant can also be made into a temporo-mandibular joint (jaw) or a finger joint. The orthopaedic bearing material can find use as an implant in any part of a body, such as the hip, knee, and extremities.

### EXAMPLE 1

This example demonstrates that a crosslinked UHMWPE containing a biocompatible hydrophobic carrier releases the carrier under cyclic loading conditions by a load activated mechanism. UHMWPE (GUR 1050) is irradiated with gamma radiation (50 KGy or 5 Mrad) and melt annealed to quench free radicals. A wear test machine such as that sold under the trade mark AMTI Ortho-POD is used for the test. The test samples are 9.5 mm (0.375 inch) in diameter and 18 mm (0.7 inch) long irradiated UHMWPE pins that are soaked in squalene at 110°C for 8 hours. The samples are sonic cleaned and dried at 50°C. The test is conducted such that the pins are dynamically loaded without articulation against a counterface to induce wear, so the results relate to the release of the biocompatible hydrophobic carrier only. A Paul loading curve, commonly used in hip stimulators, with a peak load of about 330 Newtons is applied to the pins. The cycling frequency is 1.66 Hz. A 90% bovine calf serum, diluted with EDTA and sodium azide dissolved in reverse osmosis water, is used as lubricant. The temperature of the lubricant is 37°C. During the dynamic loading test, the samples are soaked in serum without load for 2, 35, or 4 days between various test intervals.

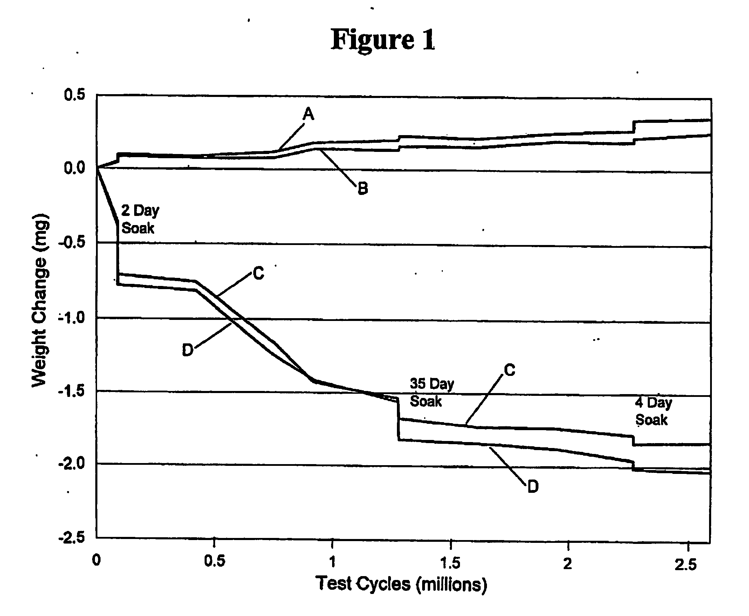

The weight change of the pins as a function of test cycles during the dynamic loading test is shown in Figure 1. Control pins, without squalene (labelled A and B), show only a slight change in weight. The test pins (labelled C and D) show significant loss of weight with load, thereby indicating that the UHMWPE samples containing squalene releases the carrier under a load activated mechanism.

### EXAMPLE 2

This example demonstrates that a crosslinked UHMWPE sample containing a biocompatible hydrophobic carrier wears at substantially same rate as UHMWPE not containing the biocompatible hydrophobic carrier. A pin-on-disk (POD) wear test method is used to evaluate weight loss of the pin. A wear test machine such as that sold under the trade mark AMTI Ortho-POD is used for the test. UHMWPE (GUR 1050) is irradiated with gamma radiation (50 KGy or 5 Mrad) and melt annealed to quench free radicals. The test samples are 9.5 mm (0.375 inch) in diameter and 18 mm (0.7 inch) long irradiated UHMWPE pins that are soaked in squalene at 110°C for 8 hours. The samples are sonic cleaned and dried at 50°C. A Paul loading curve with a peak load of about 330 Newtons is applied to the pins. The pins are moved against highly polished wrought CoCr disks in a 10 × 10 mm square pattern, synchronized with each loading cycle. The cycling frequency is 1.66 Hz. A 90% bovine calf serum, diluted with EDTA and sodium azide dissolved in reverse osmosis water, is used as lubricant. The temperature of the lubricant is 37°C. The wear test interval length is 330,000 cycles and the samples are weighed at the end of each interval.

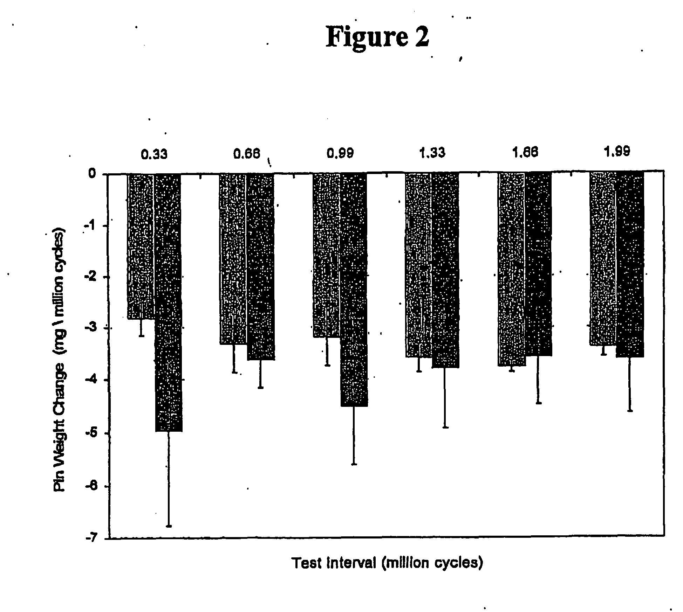

Figure 2 shows the pin weight change in mg per million cycles during the wear test. For comparison, the weight change of control pins, without squalene, is also shown. In the early stages, the test pins lose more weight; however, as the number of cycles increases, the wear rate is substantially the same as the control pins. The rate of weight change stabilizes at 1.99 million cycles.

### EXAMPLE 3

This example demonstrates that an implant according to an embodiment of the invention releases the beneficial agent, beta-carotene, which is an antioxidant and also a model compound for a beneficial agent such as a drug, by a load-activated mechanism. UHMWPE (GUR 1050) is irradiated with gamma radiation (50 KGy or 5 Mrad) and melt annealed to quench free radicals. A wear test machine such as that sold under the trade mark AMTI Ortho-POD is used to test the UHMWPE cylindrical pin samples. The samples are 9.5 mm (0.375 inch) in diameter and 18 mm (0.7 inch) long, and are soaked in a 1.0 wt. % solution of beta-carotene in squalane at 90°C for 24 hours. The samples are bright orange red in colour, indicating the presence of beta-carotene.

A Paul loading curve with a peak load of about 330 Newtons is applied to the pins. The cycling frequency is 1.66 Hz. A 90% bovine calf serum, diluted with EDTA and sodium azide dissolved in reverse osmosis water, is used as lubricant. The temperature of the lubricant is 37°C. During the test, the samples become light orange in collor, indicating that beta-carotene is being released, activated by the load.

## Claims

1. A bearing material for a medical implant or medical implant part, the bearing material comprising: (a) a matrix of crosslinked ultrahigh molecular weight polyethylene (UHMWPE), (b) a biocompatible hydrophobic carrier which is present in the bearing material in an amount of from 0.1 wt.% to 10 wt.% of the bearing material, and (c) a drug which is soluble in the biocompatible hydrophobic carrier.

2. The bearing material of claim 1, wherein the biocompatible hydrophobic carrier is a lipid.

3. The bearing material of claim 1, wherein the biocompatible hydrophobic carrier is selected from the group consisting of squalane, squalene, benzyl benzoate, fatty acids, glycerides, polyisoprenoids, cholesterol, cholesterol esters, and any combination thereof.

4. The bearing material of claim 2, wherein the lipid is selected from the group consisting of squalane, squalene, and any combination thereof.

5. The bearing material of claim 1, wherein the drug is selected from the group consisting of antibiotics, analgesics, anti-bone resorption drugs, bone growth factors, anti-cancer drugs, antioxidants, and any combination thereof.

6. A method for producing a bearing material for a medical implant or medical implant part, the method comprising:
(a) providing a raw material in consolidated form comprising ultrahigh molecular weight polyethylene,
(b) irradiating at least a portion of the raw material in consolidated form to crosslink at least a portion of the UHMWPE contained therein and so as to form a matrix of crosslinked UHMWPE,
(c) providing a solution comprising a biocompatible hydrophobic carrier and a drug which is soluble in the carrier, and
(d) contacting at least a portion of the matrix obtained in (b) with the solution to swell the polyethylene and diffuse the biocompatible hydrophobic carrier and the drug into the matrix, and
(e) drying the matrix to remove biocompatible hydrophobic carrier so that the amount that is present in the bearing material is from 0.1 wt.% to 10 wt.% of the bearing material.

7. The method of claim 6, wherein the solution is maintained at a temperature of 30°C to 150°C during step (d).

8. The method of claim 7, wherein the solution is maintained at a temperature of 50°C to 120°C during step (d).

9. The method of claim 6, wherein the matrix is contacted with the solution for 2 hours or more in step (d).

10. The method of claim 6, wherein the biocompatible hydrophobic carrier is a lipid.

11. The method of claim 6, wherein the biocompatible hydrophobic carrier is selected from the group consisting of squalane, squalene, benzyl benzoate, fatty acids, glycerides, polyisoprenoids, cholesterol, cholesterol esters, and any combination thereof.

## Patentansprüche

1. Gelenkeoberflächenmaterial für ein medizinisches Implantat oder medizinisches Implantatteil, wobei das Gelenkeoberflächenmaterial: (a) eine Matrix aus vernetztem Polyethylen mit ultrahohem Molekulargewicht (UHMWPE), (b) einen biokompatiblen hydrophoben Trägerstoff, der im Gelenkeoberflächenmaterial in einer Menge von 0,1 Gew.-% bis 10 Gew.-% des Gelenkeoberflächenmaterials vorliegt, und (c) einen Arzneistoff, der im biokompatiblen hydrophoben Trägerstoff löslich ist, umfasst.

2. Gelenkeoberflächenmaterial nach Anspruch 1, wobei der biokompatible hydrophobe Trägerstoff ein Lipid ist.

3. Gelenkeoberflächenmaterial nach Anspruch 1, wobei der biokompatible hydrophobe Trägerstoff ausgewählt ist aus der Gruppe, bestehend aus Squalan, Squalen, Benzylbenzoat, Fettsäuren, Glyceriden, Polyisoprenoiden, Cholesterol, Cholesterolestern und irgendeiner Kombination davon.

4. Gelenkeoberflächenmaterial nach Anspruch 2, wobei das Lipid ausgewählt ist aus der Gruppe, bestehend aus Squalan, Squalen und irgendeiner Kombination davon.

5. Gelenkeoberflächenmaterial nach Anspruch 1, wobei der Arzneistoff ausgewählt ist aus der Gruppe, bestehend aus Antibiotika, Analgetika, Antiknochenresorptionsmitteln, Knochenwachstumsfaktoren, Antikrebsmitteln, Antioxidationsmitteln und irgendeiner Kombination davon.

6. Verfahren zur Herstellung eines Gelenkeoberflächenmaterials für ein medizinisches Implantat oder medizinisches Implantatteil, wobei das Verfahren umfasst:
a) Bereitstellen eines Rohmaterials in konsolidierter Form, das Polyethylen mit ultrahohem Molekulargewicht umfasst,
b) Bestrahlen wenigstens eines Teils des Rohmaterials in konsolidierter Form, um wenigstens einen Teil des darin enthaltenen UHMWPE zu vernetzen und damit eine Matrix aus vernetztem UHMWPE zu bilden,
c) Bereitstellen einer Lösung, die einen biokompatiblen hydrophoben Trägerstoff und einen Arzneistoff, der im Trägerstoff löslich ist, umfasst, und
d) Inkontaktbringen wenigstens eines Teils der in (b) erhaltenen Matrix mit der Lösung, um das Polyethylen zu quellen und den biokompatiblen hydrophoben Trägerstoff und den Arzneistoff in die Matrix hinein zu diffundieren, und
e) Trocknen der Matrix, um den biokompatiblen hydrophoben Trägerstoff zu entfernen, sodass die Menge, die im Gelenkeoberflächenmaterial vorliegt, von 0,1 Gew.-% bis 10 Gew.-% des Gelenkeoberflächenmaterials beträgt.

7. Verfahren nach Anspruch 6, wobei die Lösung bei einer Temperatur von 30°C bis 150°C während Schritt (d) gehalten wird.

8. Verfahren nach Anspruch 7, wobei die Lösung bei einer Temperatur von 50°C bis 120°C während Schritt (d) gehalten wird.

9. Verfahren nach Anspruch 6, wobei die Matrix mit der Lösung für 2 Stunden oder mehr in Schritt (d) in Kontakt gebracht wird.

10. Verfahren nach Anspruch 6, wobei der biokompatible hydrophobe Trägerstoff ein Lipid ist.

11. Verfahren nach Anspruch 6, wobei der biokompatible hydrophobe Trägerstoff ausgewählt wird aus der Gruppe, bestehend aus Squalan, Squalen, Benzylbenzoat, Fettsäuren, Glyceriden, Polyisoprenoiden, Cholesterol, Cholesterolestem und irgendeiner Kombination davon.

## Revendications

1. Matériau de support pour un implant médical ou une partie d'implant médical, le matériau de support comprenant : (a) une matrice de poly-(éthylène) de masse moléculaire ultraélevée (UHMWPE) réticulé, (b) un support hydrophobe biocompatible qui est présent dans le matériau de support dans une quantité de 0,1 % en poids à 10 % en poids du matériau de support, et (c) un médicament qui est soluble dans le support hydrophobe biocompatible.

2. Matériau de support selon la revendication 1, dans lequel le support hydrophobe biocompatible est un lipide.

3. Matériau de support selon la revendication 1, dans lequel le support hydrophobe biocompatible est choisi dans le groupe constitué par le squalane, le squalène, le benzoate de benzyle, les acides gras, les glycérides, les poly(iso-prénoïdes), le cholestérol, les esters de cholestérol, et toute combinaison de ceux-ci.

4. Matériau de support selon la revendication 2, dans lequel le lipide est choisi dans le groupe constitué par le squalane, le squalène, et toute combinaison de ceux-ci.

5. Matériau de support selon la revendication 1, dans lequel le médicament est choisi dans le groupe constitué par les antibiotiques, les analgésiques, les médicaments anti-résorption osseuse, les facteurs de croissance osseuse, les médicaments anticancéreux, les antioxydants, et toute combinaison de ceux-ci.

6. Procédé de production d'un matériau de support pour un implant médical ou une partie d'implant médical, le procédé comprenant :
(a) la fourniture d'une matière première sous forme consolidée comprenant du poly(éthylène) de masse moléculaire ultraélevée,
(b) l'irradiation d'au moins une partie de la matière première sous forme consolidée pour réticuler au moins une partie de l'UHMWPE qui y est contenue et de façon à former une matrice d'UHMWPE réticulé,
(c) la fourniture d'une solution comprenant un support hydrophobe biocompatible et un médicament qui est soluble dans le support, et
(d) la mise en contact d'au moins une partie de la matrice obtenue dans (b) avec la solution pour faire gonfler le poly(éthylène) et diffuser le support hydrophobe biocompatible et le médicament dans la matrice, et
(e) le séchage de la matrice pour éliminer le support hydrophobe biocompatible de sorte que la quantité qui est présente dans le matériau de support est de 0,1 % en poids à 10 % en poids du matériau de support.

7. Procédé selon la revendication 6, dans lequel la solution est maintenue à une température de 30°C à 150°C pendant l'étape (d).

8. Procédé selon la revendication 7, dans lequel la solution est maintenue à une température de 50°C à 120°C pendant l'étape (d).

9. Procédé selon la revendication 6, dans lequel la matrice est mise en contact avec la solution pendant 2 heures ou plus dans l'étape (d).

10. Procédé selon la revendication 6, dans lequel le support hydrophobe biocompatible est un lipide.

11. Procédé selon la revendication 6, dans lequel le support hydrophobe biocompatible est choisi dans le groupe constitué par le squalane, le squalène, le benzoate de benzyle, les acides gras, les glycérides, les poly(isoprénoïdes), le cholestérol, les esters de cholestérol, et toute combinaison de ceux-ci.
